# EUROPÄISCHE PATENTSCHRIFT

(11) **EP 2 548 530 B1**
(45) Veröffentlichungstag und Bekanntmachung des Hinweises auf die Patenterteilung: **19.03.2014**
(21) Anmeldenummer: 11174505.5
(22) Anmeldetag: 19.07.2011
(51) Int. Cl.: A61C 1/08

(54) **Beleuchtungsvorrichtung für ein medizinisches, insbesondere dentales, Instrument**
Illuminating device for a medical, in particular dental instrument
Dispositif d'éclairage pour instrument médical, notamment dentaire

(43) Veröffentlichungstag der Anmeldung: 23.01.2013
(73) Patentinhaber: W & H Dentalwerk Bürmoos GmbH, 5111 Bürmoos (AT)
(72) Erfinder: Heinrich, Dr., Christoph, 5110 Oberndorf (AT); Jindra, Thomas, 5111 Bürmoos (AT)
(74) Vertreter: Benda, Ralf

(56) Entgegenhaltungen:
- EP-A1- 0 914 809
- US-A- 3 109 238

## Beschreibung

Die vorliegende Erfindung betrifft eine Beleuchtungsvorrichtung für ein medizinisches, insbesondere dentales, Instrument, insbesondere für einen Instrumentenkopf eines derartigen Instruments, mit zumindest einem Halbleiterelement, das zur Emission von elektromagnetischer Strahlung, insbesondere von Strahlung im sichtbaren Wellenlängenbereich, ausgebildet ist.

Ein derartiges Instrument in Form eines dentalen Handstücks ist zum Beispiel aus der Patentanmeldung EP 1 093 765 A2 bekannt. Gemäß einem Ausführungsbeispiel weist das Handstück einen Kopf auf, an dessen Unterseite eine Öffnung zur Abgabe eines Fluids, insbesondere von Luft oder Wasser, vorgesehen ist. Am Kopf des Handstücks, um eine Öffnung zur Aufnahme eines Behandlungswerkzeugs, ist des Weiteren eine Beleuchtungsvorrichtung mit Halbleiterelementen (LEDs) vorgesehen. Die Beleuchtungsvorrichtung mit den Halbleiterelementen ist hufeisenförmig oder C-förmig geformt und weist somit zwei freie Enden und einen Freiraum zwischen den beiden freien Enden auf. Dieser Freiraum dient zur Aufnahme der an der Unterseite des Handstückkopfs angeordneten Fluidabgabeöffnung sowie als Expansionsvolumen des sich von der Fluidabgabeöffnung kegelförmigen ausbreitenden Fluidstroms.

Durch den Freiraum zwischen den beiden freien Enden der Beleuchtungsvorrichtung ist in nachteiliger Weise ein Abschnitt am Handstück geschaffen, von dem keine Strahlung auf die Behandlungsstelle abgegeben wird, wodurch auf der Behandlungsstelle ein geringer oder schlechter ausgeleuchteter Bereich entsteht. Dies ist insbesondere nachteilig, da durch die C-förmige Ausbildung der Beleuchtungsvorrichtung auf der Behandlungsstelle eine im Wesentlichen gleichmäßige Beleuchtung geschaffen ist, an die sich das Auge des Anwenders anpasst.

Das Patent US 3,109,238 offenbart ein Instrument mit einer Beleuchtungsvorrichtung mit Glühbimen, einem, im Wesentlichen zylindrisch geformten Lichtleiter und den Lichtleiter durchsetzenden Bohrungen für Luft. Eine Antriebswelle erstreckt sich durch den Lichtleiter und ragt mit ihrem Ende aus dem Instrumentenkopf und dem Lichtleiter heraus. Das aus dem Instrumentenkopf ragende Ende der Antriebswelle ist mit einem Gewinde versehen, an das eine Gewindebuchse eines Werkzeug anschraubbar ist.

Die Patentanmeldung EP 0 914 809 A1 offenbart ein Instrument mit einem Vibrationsantrieb zur Entfernung von Zahnstein. Am Vorderende des Instruments ist ein Arm vorgesehen, an dem das Behandlungswerkzeug befestigbar ist und der die Antriebsbewegung auf das Werkzeug überträgt. Am rückwärtigen Ende des Arms sind Leuchtdioden vorgesehen, deren Licht über Lichtleiter in Richtung des Werkzeugs abgegeben wird.

Der vorliegenden Erfindung liegt daher die Aufgabe zugrunde, eine Beleuchtungsvorrichtung mit zumindest einem Halbleiterelement für ein medizinisches, insbesondere dentales, Instrument, insbesondere für einen Instrumentenkopf eines derartigen Instruments, oder ein medizinisches, insbesondere dentales, Instrument oder einen medizinischen, insbesondere dentalen, Instrumentenkopf mit einer derartigen Beleuchtungsvorrichtung unter Vermeidung der oben genannten Nachteile zu schaffen. Die Beleuchtungsvorrichtung soll insbesondere um ein mit dem Instrument oder dem Instrumentenkopf verbindbares, auf eine Behandlungsstelle einwirkendes Werkzeug oder um eine Aufnahmeöffnung für ein derartiges Werkzeug am Instrument oder Instrumentenkopf anordenbar sein, um eine wirkungsvolle Bestrahlung oder Beleuchtung der Behandlungsstelle zu erreichen, und des Weiteren eine effektive Versorgung der Behandlungsstelle mit einem oder mehreren Behandlungsfluiden zulassen.

Diese Aufgabe wird durch eine Beleuchtungsvorrichtung für einen Instrumentenkopf eines medizinischen, insbesondere dentalen, Instruments nach Anspruch 1 gelöst.

Es können am Körper der Beleuchtungsvorrichtung mehrere Öffnungen zur Abgabe von Fluid vorgesehen sein, wobei zwischen zumindest zwei Öffnungen zur Abgabe von Fluid zumindest eine optisch leitende Strahlungsabgabefläche vorgesehen ist.

Der die Aufnahme oder Öffnung umgebende Körper kann ein Instrumentenanschlussende und eine sich zwischen dem Strahlungsabgabeende und dem Instrumentenanschlussende erstreckende Mantelfläche aufweisen.

Durch das Vorsehen zumindest eines Fluidkanals oder zumindest einer Öffnung zur Abgabe von Fluid an der Beleuchtungsvorrichtung wird in vorteilhafter Weise eine gleichmäßige Strahlungs-und Fluidabgabe von dem Instrument oder dem Instrumentenkopf und insbesondere eine gleichmäßige und effektive Bestrahlung und eine gleichmäßige und effektive Fluidbeaufschlagung der Behandlungsstelle erzielt. Damit wird in vorteilhafter Weise eine gleich bleibende Ausleuchtung der Behandlungsstelle, an die sich insbesondere das Auge des Anwenders gut anpassen kann, und eine zuverlässige Kühlung der Behandlungsstelle und gegebenenfalls auch des Werkzeugs ermöglicht.

Der Begriff Instrument umfasst insbesondere mit einer Hand greifbare medizinische oder dentale Geräte, zum Beispiel gerade, pistolenförmige, gewinkelte oder gebogene Handgriffe, die im Dentalbereich oftmals als Winkelstücke bezeichnet werden, Teile von Handstücken oder Handgriffen, insbesondere einen Kopfabschnitt, der zum Beispiel mit einem davon lösbaren Griffabschnitt verbindbar ist, sowie Kupplungen, Adapter, Verbindungsstücke und Antriebseinheiten, zum Beispiel elektrische oder pneumatische Motoren. Unter der Bezeichnung Instrument werden des Weiteren sowohl schnurlose Instrumente verstanden, insbesondere mit einer austauschbaren oder aufladbaren Energiequelle, als auch Instrumente, die eine Versorgungsleitung und eine damit verbundene Steuer-, Regel- und / oder Versorgungseinheit umfassen.

Der Begriff Werkzeug umfasst insbesondere alle auf eine Behandlungsstelle einwirkenden Vorrichtungen, zum Beispiel Bohrer, Sägen, Zahnsteinentferner, Reamer, Feilen etc., aber auch elektromagnetische Strahlung, die auf eine Behandlungsstelle einwirkt, zum Beispiel Laserstrahlung, ein Partikelstrahl, zum Beispiel ein abrasive Partikel aufweisender Partikelstrahl, oder ein Fluidstrahl, zum Beispiel ein Wasserstrahl.

Das zur Emission von elektromagnetischer Strahlung ausgebildete Halbleiterelement ist vorzugsweise als Leuchtdiode (LED) ausgebildet. Die von dem Halbleiterelement emittierte Strahlung umfasst insbesondere Wellenlängen im Bereich zwischen etwa 380 nm - 780 nm, also im Wesentlichen sichtbares Licht. Die Beleuchtungsvorrichtung wird somit vorzugsweise für die Beleuchtung einer mit dem Instrument oder Instrumentenkopf behandelten Behandlungsstelle mit sichtbarem Licht verwendet. Alternativ ist das Halbleiterelement ausgebildet, Strahlung zum Aushärten lichthärtbarer Materialien abzugeben, bevorzugt Wellenlängen von in etwa 440 nm - 480 nm, oder Strahlung zum Nachweis von Karies oder Plaque zu emittieren, vorzugsweise Wellenlängen von in etwa 390 nm - 420 nm. Bevorzugt umfasst die Beleuchtungsvorrichtung mehrere Halbleiterelemente, die Strahlung mit einem ersten und einem zweiten, unterschiedlichen Wellenlängenbereiche emittieren, insbesondere die im Vorstehenden genannten Wellenlängenbereich, sowie eine Schaltvorrichtung zum wahlweisen Betrieb oder zur wahlweisen Energieversorgung oder zur wahlweisen Strahlungsemission zumindest eines Halbleiterelements, das den ersten Wellenlängenbereich emittiert, oder zumindest eines Halbleiterelements, das den zweiten Wellenlängenbereich emittiert.

Besonders vorteilhafte Ausführungsformen der im Vorstehenden beschriebenen Ausführungsbeispiele sind in den Unteransprüchen angeführt. Diese bevorzugten Ausführungsformen sind in zumindest einem im Vorstehenden beschriebenen Ausführungsbeispiel oder in zwei oder allen im Vorstehenden beschriebenen Ausführungsbeispielen implementierbar:

Der Körper der Beleuchtungsvorrichtung oder dessen (Außen-)Mantelfläche oder Außenumfang haben vorzugsweise eine zylindrische Form, insbesondere eine zylindrische Außenform, sie können jedoch auch andere Formen aufweisen, zum Beispiel eckige Formen.

Vorzugsweise weist der Körper der Beleuchtungsvorrichtung eine Oberfläche oder ein Strahlungsabgabeende auf, über welche(s) die von dem zumindest einem Halbleiterelement emittierte elektromagnetische Strahlung von der Beleuchtungsvorrichtung oder in Richtung der Behandlungsstelle abgebbar ist. Die Oberfläche oder das Strahlungsabgabeende weisen bevorzugt entweder eine oder mehrere optisch leitende, insbesondere transparente, vorzugsweise für sichtbares Licht optisch leitende, insbesondere transparente, Strahlungsabgabeflächen auf oder die Oberfläche oder das Strahlungsabgabeende bilden eine Strahlungsabgabefläche. Die Oberfläche, das Strahlungsabgabeende oder die zumindest eine Strahlungsabgabefläche sind vorzugsweise rund oder gebogen, insbesondere um die Aufnahme oder Öffnung, in welcher das Werkzeug aufnehmbar ist, gebogen, oder sie umgeben diese Aufnahme oder Öffnung ring- oder kreisförmig.

Bevorzugt weist der Körper der Beleuchtungsvorrichtung ein Instrumentenanschlussende auf. Das Instrumentenanschlussende ist ausgebildet, die Beleuchtungsvorrichtung an den Instrumentenkopf oder an das Instrument anzulagern oder anzuschließen. Vorzugsweise ist das Instrumentenanschlussende als Übergang oder Schnittstelle zum Übertragen von Medien, zum Beispiel von elektrischer Energie oder eines Fluids, zwischen dem Instrument oder dem Instrumentenkopf und der Beleuchtungsvorrichtung ausgebildet. Alternativ ist das Instrumentenanschlussende als Lager- oder Verbindungsabschnitt für Medienkontaktelemente ausgebildet, zum Beispiel für elektrische Kontakte oder für Fluidkanäle oder Fluidleitungen.

Vorzugsweise erstreckt sich die Mantelfläche des Körpers zwischen dem Strahlungsabgabeende und dem Instrumentenanschlussende des Körpers. Besonders bevorzugt sind das Strahlungsabgabeende und das Instrumentenanschlussende oder die Flächen des Körpers, die das Strahlungsabgabeende und das Instrumentenanschlussende bilden, im Wesentlichen parallel zueinander angeordnet.

Die Öffnung der Beleuchtungsvorrichtung, in welcher das Werkzeug aufnehmbar ist, hat vorzugsweise eine runde oder zylindrische Form, vorzugsweise eine runde oder zylindrisch Innenwand, sie kann jedoch auch andere Formen aufweisen, zum Beispiel eckige Formen.

Die Mantelfläche des Körpers der Beleuchtungsvorrichtung bildet vorzugsweise einen in sich geschlossenen, insbesondere (im Querschnitt) ringförmigen oder mehreckigen, Außenumfang. Die Mantelfläche ist somit nicht unterbrochen oder durchgängig oder weist kein freies Ende auf. Bevorzugt sind zum Zwecke eines kompakten Aufbaus der Beleuchtungsvorrichtung innerhalb der Mantelfläche oder deren Außenumfangs der zumindest eine Fluidkanal, der insbesondere zumindest eine an der Beleuchtungsvorrichtung vorgesehene Öffnung zur Abgabe von Fluid mit einer oder mehreren Fluidquellen verbindet, und / oder die eine oder mehreren optisch leitenden Strahlungsabgabeflächen und / oder die zumindest eine Öffnung zur Abgabe von Fluid und / oder das zumindest eine Halbleiterelement und / oder eine Mischkammer zum Mischen mehrerer Fluide angeordnet. Besonders bevorzugt sind der zumindest eine Fluidkanal und / oder die eine oder mehreren optisch leitenden Strahlungsabgabeflächen und / oder die zumindest eine Öffnung zur Abgabe von Fluid und / oder das zumindest eine Halbleiterelement und / oder eine Mischkammer zum Mischen mehrerer Fluide zwischen der Mantelfläche und der Aufnahme oder Öffnung, in welcher das Werkzeug aufnehmbar ist, angeordnet.

Die Mantelfläche erstreckt sich von einem Strahlungsabgabeende des Körpers oder von einer Oberfläche des Körpers mit einer optisch leitenden Strahlungsabgabefläche oder mit mehreren optisch leitenden Strahlungsabgabeflächen oder zwischen dem Strahlungsabgabeende und dem Instrumentenanschlussende des Körpers.

Vorzugsweise sind in der Beleuchtungsvorrichtung oder in dem Körper der Beleuchtungsvorrichtung mehrere Fluidkanäle vorgesehen, um der Beleuchtungsvorrichtung oder der Behandlungsstelle zum Beispiel unterschiedliche Fluide zuführen zu können, insbesondere ein gasförmiges Fluid, zum Beispiel Luft, und ein flüssiges Fluid, zum Beispiel Wasser oder eine wässrige Lösung. Alternativ können in den mehreren Fluidkanälen auch gleiche Fluide gefördert werden, die jedoch unterschiedliche Eigenschaften haben, zum Beispiel unterschiedliche Temperaturen, unterschiedliche Konzentrationen, unterschiedliche Drücke, unterschiedliche Volumenströme etc. Vorzugsweise können die mehreren Fluidkanäle mit unterschiedlichen Fluidquellen verbindbar oder verbunden sein.

Besonders bevorzugt münden die mehreren Fluidkanäle in eine gemeinsame Fluidmischkammer in der Beleuchtungsvorrichtung oder vereinigen sich zu einer gemeinsamen Fluidmischkammer, wobei die Fluidmischkammer mit der zumindest einen Öffnung zur Abgabe von Fluid verbunden ist. Somit sind in vorteilhafter Weise über die zumindest eine Öffnung zur Abgabe von Fluid Fluidgemische abgebbar, zum Beispiel ein Luft-Wassergemisch.

Die zumindest eine Öffnung zur Abgabe von Fluid kann vorzugsweise als Düse oder Düsenöffnung ausgebildet sein oder eine Düse oder Düsenöffnung aufweisen.

Bevorzugt sind in der Beleuchtungsvorrichtung oder in dem Körper der Beleuchtungsvorrichtung mehrere Fluidkanäle vorgesehen, wobei zumindest ein Fluidkanal eine Öffnung zur Verbindung mit einer Fluidquelle an der (Außen-)Mantelfläche oder am Außenumfang des Körpers der Beleuchtungsvorrichtung und zumindest ein Fluidkanal eine Öffnung zur Verbindung mit einer Fluidquelle an einem Instrumentenanschlussende des Körpers der Beleuchtungsvorrichtung aufweist. In vorteilhafter Weise wird damit, insbesondere wenn die Fluidkanäle sich vereinigen, eine besonders gute Vermischung der in den einzelnen Fluidkanälen geförderten Fluide erzielt.

Vorzugsweise sind der zumindest eine Fluidkanal der Beleuchtungsvorrichtung und / oder die zumindest eine Öffnung zur Abgabe von Fluid der Beleuchtungsvorrichtung über Leitungen und / oder Kanäle im Instrument oder im Instrumentenkopf mit einer oder mehreren Fluidquellen verbunden oder verbindbar.

Vorzugsweise weist die Beleuchtungsvorrichtung mehrere optisch leitende, bevorzugt transparente, insbesondere für sichtbares Licht optisch leitende, bevorzugt transparente, Strahlungsabgabeflächen und mehrere Öffnungen zur Abgabe von Fluid auf, wobei zwischen zwei Öffnungen zur Abgabe von Fluid zumindest eine Strahlungsabgabefläche angeordnet ist. Damit wird in vorteilhafter Weise eine besonders dichte, gleichmäßige und effektive Beleuchtung und Fluidbeaufschlagung der Behandlungsstelle erzielt. Bevorzugt sind zwischen drei und neun, insbesondere fünf, Strahlungsabgabeflächen vorgesehen. Bevorzugt sind zwischen drei und neun, insbesondere fünf, Öffnungen zur Abgabe von Fluid vorgesehen.

Die Anzahl der Öffnungen zur Abgabe von Fluid und der Strahlungsabgabeflächen ist entweder gleich oder unterschiedlich. Die Anzahl der Öffnungen zur Abgabe von Fluid ist gemäß einem Ausführungsbeispiel größer als die Anzahl der Strahlungsabgabeflächen. Gemäß einem alternativen Ausführungsbeispiel ist die Anzahl der Strahlungsabgabeflächen größer als die Anzahl der Öffnungen zur Abgabe von Fluid.

Gemäß einem Ausführungsbeispiel ist zwischen zwei Öffnungen zur Abgabe von Fluid eine Strahlungsabgabefläche vorgesehen. Gemäß einem alternativen Ausführungsbeispiel sind zwischen zwei Öffnungen zur Abgabe von Fluid mehrere Strahlungsabgabefläche vorgesehen. Gemäß einem anderen Ausführungsbeispiel ist zwischen zwei Strahlungsabgabeflächen eine Öffnung zur Abgabe von Fluid vorgesehen. Gemäß einem weiteren Ausführungsbeispiel sind zwischen zwei Strahlungsabgabeflächen mehrere Öffnungen zur Abgabe von Fluid vorgesehen. Gemäß einem Ausführungsbeispiel sind die Öffnungen zur Abgabe von Fluid und die Strahlungsabgabefläche alternierend angeordnet.

Gemäß einem Ausführungsbeispiel sind die zumindest eine Öffnung zur Abgabe von Fluid und die zumindest eine Strahlungsabgabefläche im Wesentlichen gleich weit von der Öffnung, in welcher das Werkzeug aufnehmbar ist, oder von einer Mittelachse dieser Öffnung beabstandet. Gemäß einem alternativen Ausführungsbeispiel sind die zumindest eine Öffnung zur Abgabe von Fluid und die zumindest eine Strahlungsabgabefläche unterschiedlich weit von der Aufnahme oder Öffnung, in welcher das Werkzeug aufnehmbar ist, oder von einer Mittelachse dieser Aufnahme oder Öffnung beabstandet. Gemäß einem Ausführungsbeispiel ist die zumindest eine Öffnung zur Abgabe von Fluid näher an der Öffnung, in welcher das Werkzeug aufnehmbar ist, oder an einer Mittelachse dieser Öffnung angeordnet als die zumindest eine Strahlungsabgabefläche. Gemäß einem alternativen Ausführungsbeispiel ist die zumindest eine Strahlungsabgabefläche näher an der Öffnung, in welcher das Werkzeug aufnehmbar ist, oder an einer Mittelachse dieser Öffnung angeordnet als die zumindest eine Öffnung zur Abgabe von Fluid.

Gemäß einem Ausführungsbeispiel sind die Öffnung zur Abgabe von Fluid und die zumindest eine Strahlungsabgabefläche nebeneinander angeordnet. Gemäß einem alternativen Ausführungsbeispiel ist die Öffnung zur Abgabe von Fluid von einer Strahlungsabgabefläche umgeben. Erfindungsgemäß sind die Öffnung zur Abgabe von Fluid und die Strahlungsabgabefläche durch einen Bereich voneinander beabstandet, der keine elektromagnetische Strahlung, insbesondere keine sichtbare elektromagnetische Strahlung, und kein Fluid abgibt.

Vorzugsweise ist die Beleuchtungsvorrichtung als Ringlicht oder ringförmige Beleuchtungsvorrichtung ausgebildet, bei denen mehrere Halbleiterelemente, die zur Emission von elektromagnetischer Strahlung ausgebildet sind, oder die eine oder mehrere Strahlungsabgabeflächen im Wesentlichen ringförmig um das an den Instrumentenkopf oder das Instrument anschließbare Werkzeug oder um die Werkzeugaufnahmeöffnung des Instrumentenkopfs oder des Instruments oder um die Werkzeughalterung des Instrumentenkopfs oder des Instruments oder um die Öffnung der Beleuchtungsvorrichtung, in welcher das Werkzeug aufnehmbar ist, angeordnet sind. Besonders bevorzugt sind auch die Öffnungen zur Abgabe von Fluid ringförmig um zumindest ein der im Vorstehenden genannten Elemente angeordnet.

Vorzugsweise sind an der Beleuchtungsvorrichtung mehrere elektrisch seriell geschaltete, mittels eines elektrischen Leiters verbundene Halbleiterelemente vorgesehen. Damit sind in vorteilhafter Weise für die elektrische Versorgung der Halbleiterelemente an der Außenseite oder Oberfläche der Beleuchtungsvorrichtung nur zwei elektrische Kontakte vorzusehen. Der elektrischen Leiter ist zum Beispiel als metallische Leiterbahn oder als metallischer Draht ausgebildet. Besonders bevorzugt ist der elektrische Leiter beabstandet von dem zumindest einen in dem Körper der Beleuchtungsvorrichtung vorgesehenen Fluidkanal angeordnet, insbesondere von einem eine Flüssigkeit leitenden Fluidkanal. Insbesondere ist zwischen dem elektrischen Leiter und dem Fluidkanal ein elektrisch isolierendes Material vorgesehen, zum Beispiel ein keramisches Material. Gemäß einem alternativen Ausführungsbeispiel sind zumindest zwei Halbleiterelemente elektrisch parallel geschaltet.

Vorzugsweise ist die Beleuchtungsvorrichtung aus mehreren Schichten, insbesondere aus mehreren Schichten unterschiedlicher Materialien, aufgebaut. Die Materialien, aus denen eine oder mehrere der Schichten aufgebaut sind, umfassen zum Beispiel Glas, Kunststoff, Keramik oder Metall.

Bevorzugt dienen das Glas und / oder der Kunststoff zum optischen Leiten der von dem zumindest einem Halbleiterelement emittierten elektromagnetischen Strahlung durch die Beleuchtungsvorrichtung und / oder an die zumindest eine Strahlungsabgabefläche der Beleuchtungsvorrichtung. Besonders bevorzugt besteht die zumindest eine Strahlungsabgabefläche oder zumindest ein Teil des Strahlungsabgabeendes der Beleuchtungsvorrichtung oder jene Schicht, die eine Strahlungsabgabefläche oder zumindest ein Teil des Strahlungsabgabeendes bildet, aus Glas oder Kunststoff. Besonders bevorzugt emittiert das zumindest eine Halbleiterelement sichtbare Strahlung und sind das Glas und / oder der Kunststoff, die die sichtbare Strahlung optisch leiten, für sichtbare Strahlung transparent.

Vorzugsweise umfassen jene Schicht oder Schichten ein keramisches Material und / oder Kunststoff, in welchen das zumindest eine Halbleiterelement und / oder ein elektrischer Leiter zur Versorgung des zumindest einen Halbleiterelements mit elektrischer Energie vorgesehen ist / sind. Bevorzugt besteht die Schicht, welche einen elektrischen Leiter aufweist, aus zumindest zwei keramischen Teilschichten, zwischen denen der elektrische Leiter angeordnet ist. Besonders bevorzugt weist der zwischen den keramischen Teilschichten angeordnete Leiter keinen elektrisch isolierenden Außenmantel oder keine elektrische Außenisolierung auf. Das keramische Material umfasst zum Beispiel Aluminiumoxid-, Siliciumnitrid- oder Aluminiumnitridkeramiken.

Vorzugsweise umfasst zumindest eine der Schichten metallisches Material und / oder Kunststoff und / oder Glas, insbesondere jene Schicht, die das Strahlungsabgabeende der Beleuchtungsvorrichtung bildet oder jene Schicht, an welcher die zumindest eine optisch leitenden Strahlungsabgabefläche vorgesehen ist. Das metallische Material umfasst zum Beispiel Stahl. Der Kunststoff umfasst zum Beispiel Polymethylmethacrylat (PMMA).

Ein Ausführungsbeispiel einer Beleuchtungsvorrichtung umfasst eine keramische Schicht, an welcher das zumindest eine Halbleiterelement und / oder der elektrischer Leiter zur Versorgung des zumindest einen Halbleiterelements vorgesehen ist / sind, und eine damit verbundene metallische Schicht, in welcher zumindest ein optischer Leiter aus Glas und / oder zumindest eine Strahlungsabgabefläche aus Glas zum Leiten der von dem Halbleiterelement emittierten elektromagnetischen insbesondere sichtbaren, Strahlung angeordnet ist / sind. Vorzugsweise ist das Glas in die metallische Schicht eingeschmolzen.

Ein alternatives Ausführungsbeispiel einer Beleuchtungsvorrichtung umfasst eine keramische Schicht, an welcher das zumindest eine Halbleiterelement und / oder der elektrischer Leiter zur Versorgung des zumindest einen Halbleiterelements vorgesehen ist / sind, und eine damit verbundene Glasschicht zum Leiten der von dem Halbleiterelement emittierten elektromagnetischen, insbesondere sichtbaren Strahlung. Dieses Ausführungsbeispiel weist keine metallische Schicht auf.

Ein anderes Ausführungsbeispiel einer Beleuchtungsvorrichtung umfasst eine erste metallische Schicht, an welcher das zumindest eine Halbleiterelement und / oder der elektrischer Leiter zur Versorgung des zumindest einen Halbleiterelements vorgesehen ist / sind, gegebenenfalls von der metallischen Schicht durch ein elektrisches Isolationsmittel getrennt, und eine damit verbundene zweite metallische Schicht, in welcher zumindest ein optischer Leiter aus Glas und / oder zumindest eine Strahlungsabgabefläche aus Glas angeordnet ist / sind oder eine damit verbundene Glasschicht, so wie dies im Vorstehenden beschrieben ist.

Vorzugsweise weist die Beleuchtungsvorrichtung mehrere Halbleiterelemente auf, wobei jedem Halbleiterelement ein eigener optischer Strahlungsleiter zugeordnet ist, der derart angeordnet ist, dass er die von den Halbleiterelementen emittierte elektromagnetische Strahlung in Richtung des Strahlungsabgabeendes der Beleuchtungsvorrichtung oder in Richtung der zumindest einen optisch leitenden Strahlungsabgabefläche leitet. Die Strahlungsleiter erstrecken sich zum Beispiel von den Halbleiterelementen in Richtung des Strahlungsabgabeendes oder der Strahlungsabgabefläche. Zur weiteren Verbesserung der Strahlungs-/Lichtübertragung können die Strahlungsleiter bevorzugt beschichtet sein. Besonders bevorzugt bildet zumindest ein Ende eines Strahlungsleiters zumindest einen Teil der Strahlungsabgabefläche der Beleuchtungsvorrichtung oder zumindest einen Teil des Strahlungsabgabeendes der Beleuchtungsvorrichtung. Die Strahlungsleiter umfassen zum Beispiel einen Glaskörper, Glasstab, Glasfaserkörper, Glasfaserstab, Kunststoffkörper oder Kunststoffstab. Besonders bevorzugt sind die optischen Strahlungsleiter voneinander beabstandet, insbesondere durch ein Material voneinander getrennt, das keine elektromagnetische Strahlung, insbesondere keine sichtbare elektromagnetische Strahlung, abgibt oder das nicht transparent ist, insbesondere nicht für Licht transparent ist.

Vorzugsweise ist das zumindest eine Halbleiterelement in einer, insbesondere hermetisch verschlossenen, Kammer im Körper der Beleuchtungsvorrichtung angeordnet. Bevorzugt ist die Kammer derart hermetisch verschlossen, dass keine Partikel und / oder Wasserdampf und / oder Flüssigkeiten in die Kammer eindringen. Besonders bevorzugt ist die Kammer derart hermetisch verschlossen, dass sie mehreren Reinigungs- oder Sterilisationsvorgängen widersteht, so dass in den Vorgängen verwendete Medien, wie Reinigungsmittel oder Wasserdampf, nicht in die Kammer eindringen. Bevorzugt ist zumindest ein Teil der Kammer oder die den Innenraum der Kammer umgebende Kammerwand durch ein Material gebildet, welches für die von dem Halbleiterelement emittierte Strahlung transparent ist und / oder diese Strahlung leitet.

Vorzugsweise ist die Kammer oder die den Innenraum der Kammer umgebende Kammerwand durch mehrere Schichten der Beleuchtungsvorrichtung gebildet, die miteinander verbunden sind, zum Beispiel durch verschmelzen, einschmelzen, löten, verschweißen, verkleben, etc. Gemäß einem Ausführungsbeispiel ist die Kammerwand durch eine Keramikschicht und eine damit verbundene Metallschicht gebildet. Besonders bevorzugt ist in der Metallschicht ein optischer Strahlungsleiter angeordnet, der insbesondere auch einen Teil der Kammerwand bildet. Gemäß einem anderen Ausführungsbeispiel ist die Kammerwand durch eine Keramikschicht und eine damit verbundene Glasschicht gebildet. Gemäß einem weiteren Ausführungsbeispiel ist die Kammerwand durch eine Keramikschicht und eine Metallschicht und eine Glasschicht, die miteinander verbunden sind, gebildet.

Vorzugsweise ist in der Kammer neben dem Halbleiterelement zumindest ein zusätzliches Bauteil vorgesehen. Gemäß einem Ausführungsbeispiel umfasst das zusätzliche Bauteil ein Element zur Umwandlung der Wellenlänge der vom Halbleiterelement abgestrahlten Strahlung, zum Beispiel einen Konverter, eine Konversionsfolie oder eine Konversionspaste. Gemäß einem anderen Ausführungsbeispiel umfasst das zusätzliche Bauteil einen elektrischen Leiter zur Versorgung des Halbleiterelements mit elektrischer Energie. Gemäß einem anderen Ausführungsbeispiel umfasst das zusätzliche Bauteil ein optisches Element, zum Beispiel einen Reflektor, insbesondere zum Lenken der vom Halbleiterelement abgestrahlten Strahlung in Richtung der Strahlungsabgabefläche und / oder eine Linse zum Bündeln der vom Halbleiterelement abgestrahlten Strahlung und / oder einen optischen Filter.

Bevorzugt schließt an die, insbesondere hermetisch verschlossene, Kammer ein optischer Strahlungsleiter an, der derart angeordnet ist, dass er die von den Halbleiterelementen emittierte elektromagnetische Strahlung in Richtung des Strahlungsabgabeendes der Beleuchtungsvorrichtung oder in Richtung der zumindest einen optisch leitenden Strahlungsabgabefläche leitet, so wie er insbesondere im Vorstehenden beschrieben ist. Alternativ bildet der optische Strahlungsleiter oder eine Fläche des optischen Strahlungsleiters einen Teil der den Innenraum der Kammer umschließenden Kammerwand.

Vorzugsweise umfasst die Beleuchtungsvorrichtung eine Steuervorrichtung zur alternierenden Abgabe der elektromagnetischen Strahlung und des Fluids vom Strahlungsabgabeende der Beleuchtungsvorrichtung. Gemäß einem Ausführungsbeispiel ist die Steuervorrichtung ausgebildet, abwechselnd einen oder mehrere Strahlungspulse und anschließend einen Fluidpuls von der Beleuchtungsvorrichtung abzugeben, die im Wesentlichen zeitlich aufeinander folgen. Gemäß einem alternativen bevorzugten Ausführungsbeispiel ist die Steuervorrichtung ausgebildet, abwechselnd einen oder mehrere Fluidpulse und anschließend einen Strahlungspuls von der Beleuchtungsvorrichtung abzugeben, die im Wesentlichen zeitlich aufeinander folgen.

Die Steuervorrichtung umfasst bevorzugt ein oder mehrere auf die Lichtabgabevorrichtung oder auf die Kühlmedienabgabevorrichtung einwirkende Steuer- oder Stellelemente und insbesondere einen damit verbundenen Mikrocontroller zur Ansteuerung der Steuer- oder Stellelemente. Besonders bevorzugt weist die Steuervorrichtung ein in einer Medienleitung eines Kühlmediums angeordnetes Steuerventil auf, insbesondere ein Magnetventil, das vom Mikrocontroller zum Öffnen oder Schließen durch Steuersignale periodisch ansteuerbar ist. Die Pulsrate der Steuersignale oder des Steuerventils und somit auch der Kühlmedienabgabe beträgt zumindest in etwa 25 Hz, bevorzugt mehr als etwa 50 Hz, besonders bevorzugt etwa 75 Hz.

Bevorzugt weist die Steuervorrichtung des Weiteren ein elektrisches oder elektronisches Schaltelement zur gepulsten Energieversorgung (Versorgung mit elektrischem Strom) des zumindest einen Halbleiterelements auf. Die Pulsrate der Beleuchtungsvorrichtung liegt bevorzugt über der Flimmerfrequenz des menschlichen Auges und beträgt zumindest in etwa 25 Hz, besonders bevorzugt mehr als etwa 50 Hz, so dass für den Anwender der Eindruck einer kontinuierlichen Lichtabgabe entsteht.

Vorzugsweise ist die Beleuchtungsvorrichtung lösbar mit dem medizinischen, insbesondere dentalen, Instrument oder Instrumentenkopf verbindbar. Die lösbare Verbindung erfolgt zum Beispiel mittels einer Steck-, Klemm- oder Schraubverbindung. Vorzugsweise ist an der Beleuchtungsvorrichtung ein Anschlag, zum Beispiel ein Flansch, vorgesehen, der ausgebildet ist, an einem Gegenanschlag des Instrumentenkopfs anzuliegen, sobald die Beleuchtungsvorrichtung ihre vornestimmte Position am Instrumentenkopf einnimmt.

Vorzugsweise ist an dem Instrumentenkopf eine Aufnahme, zum Beispiel eine Rücksprung, vorgesehen, in dem die Beleuchtungsvorrichtung, insbesondere lösbar, aufnehmbar ist. Bevorzugt ist die Aufnahme ausgebildet, Fluid an die Beleuchtungsvorrichtung zu übertragen. Besonders bevorzugt ist diese Aufnahme mit zumindest einer Fluidleitung des Instrumentenkopfs oder des Instruments verbunden oder mündet eine Fluidleitung in die Aufnahme, so dass Fluid von einer Fluidquelle über die Fluidleitung und über oder durch die Aufnahme zur Beleuchtungsvorrichtung oder zu dem zumindest einen Fluidkanal der Beleuchtungsvorrichtung oder zur Fluidabgabeöffnung der Beleuchtungsvorrichtung förderbar ist. Alternativ oder zusätzlich dient zumindest ein Teil der Aufnahme als Fluidkanal, welcher der Beleuchtungsvorrichtung ein Fluid zuführt oder bildet zumindest ein Teil der Aufnahme gemeinsam mit der Beleuchtungsvorrichtung, zum Beispiel einer Ausnehmung der Beleuchtungsvorrichtung, zumindest einen Abschnitt des Fluidkanals der Beleuchtungsvorrichtung.

Gemäß einem Ausführungsbeispiel ist die Beleuchtungsvorrichtung elektrisch mit einem in dem medizinischen, insbesondere dentalen, Instrument angeordneten Generator verbunden, so dass die Beleuchtungsvorrichtung, insbesondere das zumindest eine Halbleiterelement, mit von dem Generator erzeugter elektrischer Energie versorgbar ist. Der Generator ist zur Erzeugung der elektrischen Energie vorzugsweise durch ein Antriebselement des Instruments, das ausgebildet ist, die Werkzeughaltevorrichtung oder ein mit dem Instrument verbindbares Werkzeug in Bewegung zu versetzen, antreibbar, zum Beispiel durch eine Antriebswelle. Alternativ ist der Generator durch ein Fluid, insbesondere durch ein Antriebsfluid für die Werkzeughaltevorrichtung oder ein mit dem Instrument verbindbares Werkzeug, antreibbar.

Vorzugsweise ist der Beleuchtungsvorrichtung ein elektrischer oder elektronischer Steuer-oder Schaltkreis zugeordnet oder es ist in dem medizinischen, insbesondere dentalen, Instrument ein elektrischer oder elektronischer Steuer- oder Schaltkreis vorgesehen, der ausgebildet ist, zumindest einen Parameter der elektrischen Energie zur Versorgung des zumindest einen Halbleiterelements, insbesondere die elektrische Spannung, an das Halbleiterelement anzupassen. Mittels des Steuer-oder Schaltkreises ist alternativ oder zusätzlich zum Beispiel auch die Helligkeit der von dem Halbleiterelement emittierten Strahlung einstellbar oder es ist aus mehreren Halbleiterelementen wahlweise ein Halbleiterelement mit einer bestimmten Eigenschaft, zum Beispiel mit einem spezifischen Wellenlängenbereich, wie im Vorstehenden bereits beschrieben, betreibbar oder mit Energie versorgbar.

Die Erfindung wird nachfolgend anhand bevorzugter Ausführungsbeispiele und Bezug nehmend auf die beigefügten Zeichnungen erläutert:
Figur 1 zeigt ein Ausführungsbeispiel eines medizinischen, insbesondere dentalen, Instruments mit einer Beleuchtungsvorrichtung mit zumindest einem Halbleiterelement zur Emission von elektromagnetischer, insbesondere sichtbarer, Strahlung und zumindest einem in der Beleuchtungsvorrichtung vorgesehenen Fluidkanal, der zumindest eine am Strahlungsabgabeende der Beleuchtungsvorrichtung vorgesehene Öffnung zur Abgabe von Fluid mit einer oder mehreren Fluidquellen verbindet.
Figur 2 zeigt ein Ausführungsbeispiel eines Strahlungsabgabeendes einer Beleuchtungsvorrichtung mit zumindest einem Halbleiterelement, wobei das Strahlungsabgabeende mehrere Strahlungsabgabeflächen und mehrere Öffnung zur Abgabe von Fluid aufweist.
Figur 3 einen Querschnitt durch die Beleuchtungsvorrichtung der Figur 2 entlang der Linie "A - A".
Figur 4 zeigt den in der Figur 3 mit "B" bezeichnet Ausschnitt in vergrößertem Maßstab mit einem Halbleiterelement und einem dem Halbleiterelement zugeordneten optischen Leiter.
Figur 5 zeigt einen Längsschnitt durch die Beleuchtungsvorrichtung der Figur 3 entlang der Linie "C - C" sowie die elektrische Versorgung der Halbleiterelemente.
Figur 6 zeigt einen Querschnitt durch eine alternative Ausführungsform einer Beleuchtungsvorrichtung mit zumindest einem Halbleiterelement und zumindest einer Öffnung zur Abgabe von Fluid.
Figur 7 zeigt ein weiteres Ausführungsbeispiel einer Kammer einer Beleuchtungsvorrichtung mit einem Halbleiterelement und einem dem Halbleiterelement zugeordneten optischen Leiter.
Figur 8 zeigt ein alternatives Ausführungsbeispiel eines Strahlungsabgabeendes einer Beleuchtungsvorrichtung mit zumindest einem Halbleiterelement, wobei das Strahlungsabgabeende eine Strahlungsabgabefläche aufweist, in der mehrere Öffnungen zur Abgabe von Fluid angeordnet sind, wobei dieses Ausführungsbeispiel nicht Gegenstand des Anspruchs 1 ist.
Figur 9 zeigt ein Ausführungsbeispiel eines Instrumenten- oder Winkelstückkopfs mit einer damit lösbar verbundenen Beleuchtungsvorrichtung mit zumindest einem Halbleiterelement und zumindest einer Öffnung zur Abgabe von Fluid sowie in vergrößerten Ausschnitten ein Halbleiterelement der Beleuchtungsvorrichtung und einen Fluidkanal der Beleuchtungsvorrichtung.
Figur 10 zeigt ein alternatives Ausführungsbeispiel einer Beleuchtungsvorrichtung mit einem Ringkanal für ein Fluid an der Mantelfläche.
Figur 11 zeigt ein alternatives Ausführungsbeispiel eines Instrumenten- oder Winkelstückkopfs mit einer damit unlösbar verbundenen Beleuchtungsvorrichtung.
Figur 12 zeigt ein alternatives Ausführungsbeispiel einer Beleuchtungsvorrichtung, deren Körper aus Kunstharz besteht, in welchem das zumindest eine Halbleiterelement vergossen ist.
Figur 13 zeigt ein alternatives Ausführungsbeispiel einer Beleuchtungsvorrichtung mit zwei freien Enden.
Figur 14 zeigt ein alternatives Ausführungsbeispiel einer Beleuchtungsvorrichtung, deren Körper aus zwei Teilringen besteht.

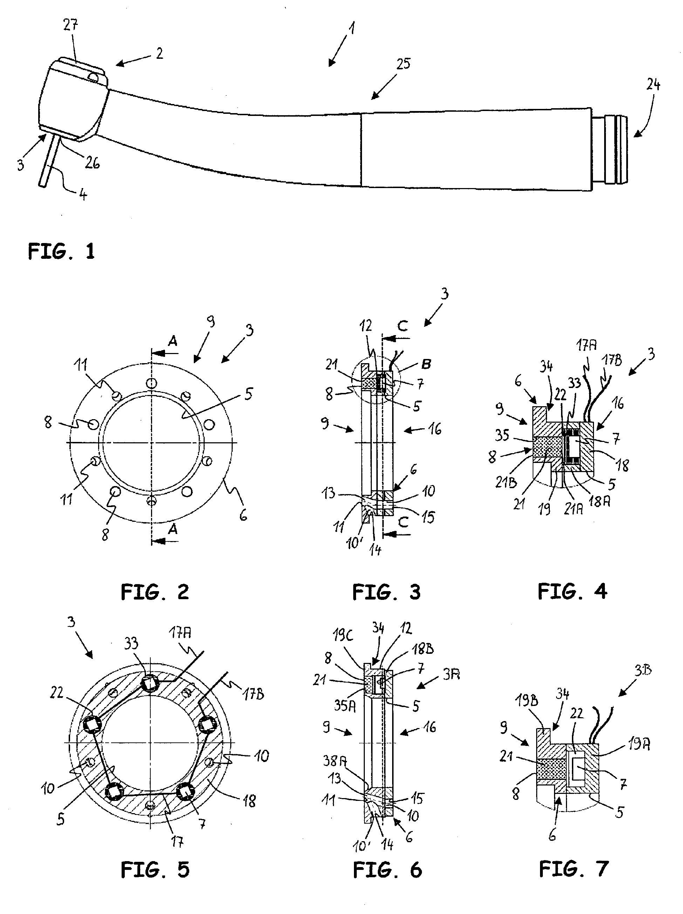

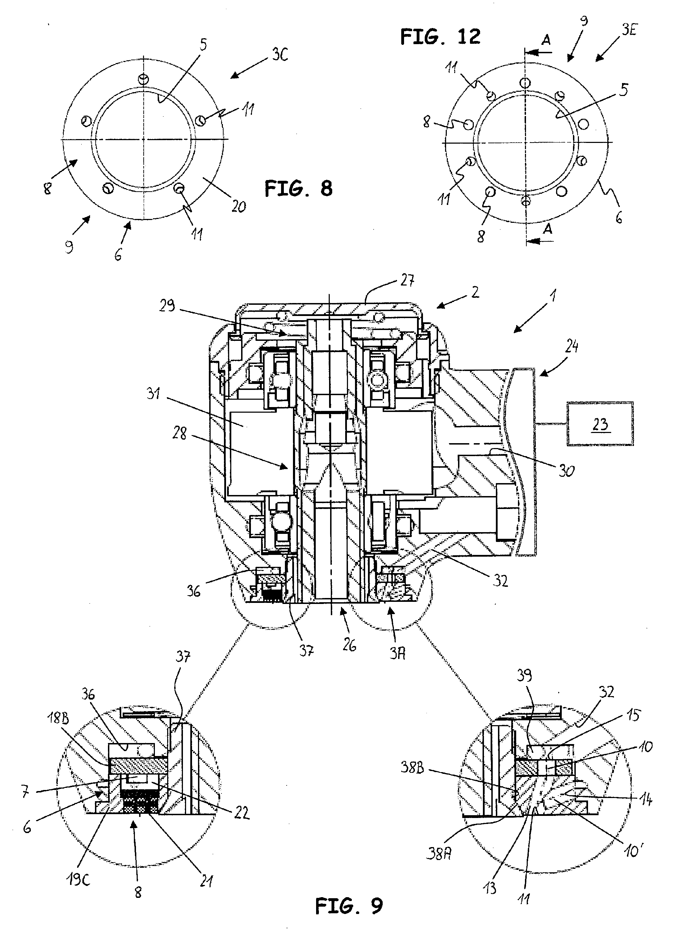

Das in den Figuren 1 und 9 dargestellte medizinische, insbesondere dentale, Instrument 1 ist als längliches, rohrförmiges Instrument 1 oder Handstück ausgebildet, das an einem Ende einen Anschluss 24, zum lösbaren Verbinden zum Beispiel an eine Steuervorrichtung, an eine Antriebseinheit, an eine Energiequelle und / oder an eine Fluidquelle, insbesondere eine Wasser- und / oder Druckluftquelle, aufweist. Das Instrument 1 umfasst einen gebogenen oder einen zwei gewinkelt zueinander angeordnete Abschnitte aufweisenden Griffteil 25 und einen daran anschließenden Instrumentenkopf 2. Am Instrumentenkopf 2 ist eine Werkzeugöffnung 26 vorgesehen, durch die ein Werkzeug 4 zum Einwirken auf eine Behandlungsstelle lösbar in den Instrumentenkopf 2 einbringbar ist. Im Instrumentenkopf 2 ist eine lösbare Werkzeughaltevorrichtung 28, zum Beispiel eine Spannzange, angeordnet, welche das Werkzeug 4 lösbar am Instrumentenkopf 2 befestigt. Die Werkzeugöffnung 26 ist seitlich am Instrumentenkopf 2 angeordnet, so dass das Werkzeug 4 gewinkelt zum Griffteil 25 oder dessen Längsachse aus dem Instrumentenkopf 2 ragt. An dem der Werkzeugöffnung 26 gegenüberliegenden Ende des Instrumentenkopfs 2 ist ein Drucktaster 27 vorgesehen, der mit einer im Instrumentenkopf 2 angeordneten Werkzeuglösevorrichtung 29 zusammenwirkt, um das Werkzeug 4 aus dem Instrumentenkopf 2 oder der Werkzeughaltevorrichtung 28 frei zu geben. Selbstverständlich kann das Instrument 1 auch andere bekannte Formen aufweisen, zum Beispiel pistolenförmig oder gerade ausgebildet sein.

Von der Anschlussvorrichtung 24 erstrecken sich zum Beispiel eine Vorrichtung zur Übertragung von Antriebsenergie auf die Werkzeughaltevorrichtung 28, insbesondere eine Welle oder eine Fluidleitung, eine oder mehrere Fluid- oder Medienleitungen 32, zum Beispiel eine oder mehrere Medienleitungen für (Kühl-)Wasser oder (Kühl-)Druckluft, Lichtleiter und / oder elektrische Versorgungs- oder Steuerleitungen durch das Instrument 1. Gemäß der Figur 9 fördert eine Fluidleitung 30 ein Antriebsfluid, zum Beispiel Druckluft, zu einer Antriebseinheit in Form eines in Drehung versetzbaren Drehteils 31, insbesondere eines Laufrads. Das Drehteil 31 ist mit der Werkzeughaltevorrichtung 28 verbunden, um diese und ein darin aufgenommenes Werkzeug 4 in Bewegung zu versetzen.

An dem Instrumentenkopf 2, insbesondere an dem Ende des Instrumentenkopfs 2 mit der Werkzeugöffnung 26, ist eine Beleuchtungsvorrichtung 3, 3A vorgesehen. Die Beleuchtungsvorrichtung 3, 3A ist insbesondere um die Werkzeugöffnung 26 angeordnet oder umgibt diese ringförmig, so dass die Beleuchtungsvorrichtung 3, 3A als Ringlicht ausgebildet ist.

Die Figuren 2 - 4 zeigen ein Ausführungsbeispiel einer Beleuchtungsvorrichtung 3: Die Beleuchtungsvorrichtung 3 weist ein Strahlungsabgabeende 9 und ein im Wesentlichen parallel dazu angeordnetes Instrumentenanschlussende 16 auf. Vom Strahlungsabgabeende 9 emittiert die Beleuchtungsvorrichtung 3 elektromagnetische Strahlung, insbesondere sichtbare elektromagnetische Strahlung. Das Instrumentenanschlussende 16 dient zum Anlagern oder Verbinden der Beleuchtungsvorrichtung 3 an das Instrument 1. Zwischen den beiden Enden 9, 16 erstreckt sich ein Körper 6 der Beleuchtungsvorrichtung 3 mit einer (Außen-)Mantelfläche 12. Die Mantelfläche 12 verbindet somit die beiden Enden 9, 16. Die Mantelfäche 12 ist in sich geschlossen oder weist einen geschlossenen, insbesondere zylindrischen, Außenumfang auf.

Der Körper 6 der Beleuchtungsvorrichtung 3 weist eine, insbesondere zentrale, Bohrung oder Aufnahme oder Öffnung 5 auf. Die Öffnung 5 ist fluchtend an die Werkzeugöffnung 26 angeschlossen oder umgibt die Werkzeugöffnung 26. In der Aufnahme oder Öffnung 5 ist das Werkzeug 4 aufnehmbar oder durch die Aufnahme oder Öffnung 5 ist das Werkzeug 4 in den Instrumentenkopf 2 einbringbar oder daraus lösbar. Wie aus der Figur 2 erkennbar ist, kann die Bohrung oder Öffnung 5 zylindrisch ausgebildet sein.

An oder in dem Körper 6 ist ein Halbleiterelement 7 vorgesehen, das zur Emission von elektromagnetischer Strahlung, insbesondere von sichtbarer Strahlung (Licht), ausgebildet ist. Das Halbleiterelement 7 ist zum Beispiel als Leuchtdiode (LED) oder als Dice ausgebildet. Zumindest ein Teil der von dem Halbleiterelement 7 erzeugten Strahlung wird über eine oder mehrere optisch leitende, insbesondere transparente, Licht- oder Strahlungsabgabeflächen 8 an dem Strahlungsabgabeende 9 der Beleuchtungsvorrichtung 3 oder an einer Oberfläche der Beleuchtungsvorrichtung 3 abgegeben.

In dem Körper 6 der Beleuchtungsvorrichtung 3 ist des Weiteren zumindest ein Fluidkanal 10, 10' vorgesehen, der von dem Strahlungsabgabeende 9 oder der Oberfläche mit der Strahlungsabgabefläche 8 durch zumindest einen Teil der Beleuchtungsvorrichtung 3 verläuft. Der Fluidkanal 10, 10' endet mit einer Öffnung 11 zur Abgabe von Fluid am Strahlungsabgabeende 9 oder der Oberfläche mit der Licht- oder Strahlungsabgabefläche 8. Der zumindest eine Fluidkanal 10, 10' ist mit der Fluidleitung 32 verbunden (siehe Figur 9) und ausgebildet, zumindest ein Fluid aus der Fluidleitung 32 durch die Beleuchtungsvorrichtung 3 bis zur Öffnung 11 zu leiten. Aus der Öffnung 11 wird das Fluid an die Umgebung abgegeben, zum Beispiel auf die Behandlungsstelle und / oder das Werkzeug 4.

Der zumindest eine Fluidkanal 10, 10', das zumindest eine Halbleiterelement 7, die Öffnung 11 und die Licht- oder Strahlungsabgabefläche 8 sind innerhalb der in sich geschlossenen Mantelfläche 12 des Körpers 6 oder des in sich geschlossenen Außenumfangs der Mantelfläche 12 angeordnet.

Das Ausführungsbeispiel gemäß Figur 2 zeigt ein Strahlungsabgabeende 9 einer Beleuchtungsvorrichtung 3 mit mehreren, insbesondere jeweils fünf, Öffnungen 11 zur Abgabe von Fluid und Licht- oder Strahlungsabgabeflächen 8. Die Öffnungen 11 und die Strahlungsabgabeflächen 8 sind jeweils abwechselnd angeordnet. Zwischen zwei Öffnungen 11 ist eine Strahlungsabgabefläche 8 bzw. zwischen zwei Strahlungsabgabeflächen 8 ist eine Öffnung 11 vorgesehen. Die Öffnungen 11 und die Strahlungsabgabeflächen 8 umgeben die Aufnahme oder Öffnung 5 im Wesentlichen kreisförmig. Die Öffnungen 11 sind etwas näher an der Aufnahme oder Öffnung 5 positioniert als die Strahlungsabgabeflächen 8. Die Öffnungen 11 und die Strahlungsabgabeflächen 8 sind voneinander beabstandet. Die Abschnitte oder Flächen des Strahlungsabgabeendes 9 zwischen den Öffnungen 11 und den Strahlungsabgabeflächen 8 sind nicht dazu ausgebildet, Fluid und / oder Strahlung abzugeben, insbesondere sind diese Flächen im Wesentlichen nicht optisch leitend oder nicht transparent für sichtbares Licht. Die Flächen sind zum Beispiel aus Metall, insbesondere aus Stahl hergestellt. Selbstverständlich ist jedoch auch eine andere Anzahl und / oder Anordnung der Öffnungen 11 und / oder der Strahlungsabgabeflächen 8 möglich.

Aus der Figur 3 ist zu erkennen, dass die Beleuchtungsvorrichtung 3 zumindest zwei Fluidkanäle 10, 10' aufweist. Vorzugsweise sind diese beiden Fluidkanäle 10, 10' mit unterschiedlichen Fluidleitungen 32 des Instruments 1 verbunden, insbesondere mit unterschiedlichen Fluidquellen, zum Beispiel mit einer Wasserquelle und einer Druckluftquelle. Der Fluidkanal 10' weist eine Öffnung 14 zur Verbindung mit einer Fluidquelle an der Mantelfläche 12 des Körpers 6 auf, der Fluidkanal 10 eine Öffnung 15 zur Verbindung mit einer Fluidquelle an dem Instrumentenanschlussende 16 des Körpers 6 auf.

Vorzugsweise vereinigen sich die beiden Fluidkanäle 10, 10' zu einer Fluidmischkammer 13 oder münden in eine Fluidmischkammer 13, in welcher sich die Fluide aus den beiden Fluidkanälen 10, 10' vermischen. Die Fluidmischkammer 13 und über die Fluidmischkammer 13 auch die beiden Fluidkanäle 10, 10' sind mit zumindest einer Öffnung 11 zur Abgabe von Fluid verbunden, über welche die beiden, bevorzugt vermischten, Fluide oder das Fluidgemisch von der Beleuchtungsvorrichtung 3, insbesondere von deren Strahlungsabgabeende 9, abgegeben werden.

Insbesondere aus der Figur 4 ist ersichtlich, dass das zumindest eine Halbleiterelement 7 in einer, insbesondere hermetisch verschlossenen, Kammer 22 aufgenommen ist. Bei Vorhandensein mehrerer Halbleiterelemente 7 sind vorzugsweise auch mehrere voneinander getrennte Kammern 22 vorgesehen, insbesondere ist in jeder Kammer 22 nur jeweils ein Halbleiterelement 7 aufgenommen (siehe Figur 5). Die Kammer 22 schützt das Halbleiterelement 7 vor Kontamination, insbesondere vor Partikeln oder flüssigen, gas- oder dampfförmigen Verunreinigungen.

Die Kammer 22 ist innerhalb der Mantelfläche 12 des Körpers 6 angeordnet. Die Kammer 22 oder die Innenwände der Kammer 22 werden vorzugsweise durch mehrere Schichten 18, 19, aus denen der Körper 6 der Beleuchtungsvorrichtung 3 besteht, gebildet.

In der Kammer 22 ist bevorzugt neben dem Halbleiterelement 7 zumindest ein zusätzliches Bauteil vorgesehen. Wie insbesondere in der Figur 4 zu erkennen ist, umfasst das zusätzliche Bauteil ein Element 33 zur Umwandlung der Wellenlänge der vom Halbleiterelement abgestrahlten Strahlung, insbesondere zum Umwandeln in weißes Licht, zum Beispiel einen Konverter, insbesondere eine Konversionsfolie oder eine Konversionspaste. Solche Konverter können Farbkonversionsmaterialien enthalten, beispielsweise Lumineszenzfarbstoffe, die insbesondere durch blaues Licht zum Leuchten angeregt werden, wodurch langwelligeres, gelbes Licht abgegeben wird. Da nicht das gesamte blaue Licht umgewandelt wird, gibt die resultierende additive Mischung der Spektralfarben ein weißes Licht. Als Farbkonversionsmaterialien können beispielsweise Orthosilikate oder YAG Farbstoffe eingesetzt werden, die Farbkonversionspigmente können insbesondere in einem organischen Trägermaterial, bevorzugt transparenter Epoxidharz oder silikonbasierte Trägermaterialen eingebettet sein.

Die Beleuchtungsvorrichtung 3 weist des Weiteren zumindest einen optischen Strahlungsleiter 21 auf, der derart angeordnet ist, dass er von dem Halbleiterelement 7 emittierte elektromagnetische Strahlung in Richtung des Strahlungsabgabeendes 9 und / oder der Licht- oder Strahlungsabgabefläche 8 der Beleuchtungsvorrichtung 3 leitet. Sind mehrere Halbleiterelement 7 vorgesehen, so können auch mehrere Strahlungsleiter 21 vorhanden sein, vorzugsweise ist jedem Halbleiterelement 7 ein eigener Strahlungsleiter 21 zugeordnet. Der Strahlungsleiter 21 weist zwei Enden 21 A, 21 B auf, wobei ein erstes Ende 21 A einem Halbleiterelement 7 zugewandt ist. Das erste Ende 21 A kann an die Kammer 22 anschließen oder einen Teil der Begrenzung der Kammer 22 bilden, insbesondere einen Teil der Begrenzungswand der Kammer 22. Das zweite Ende 21 B des Strahlungsleiters 21 weist in Richtung des Strahlungsabgabeendes 9 oder bildet zumindest einen Teil der Licht- oder Strahlungsabgabefläche 8 der Beleuchtungsvorrichtung 3. Der Strahlungsleiter 21 ist zum Beispiel als Zylinder, Stab (siehe Figur 4) oder flache Scheibe (siehe Figur 6) ausgebildet. Der Strahlungsleiter 21 ist insbesondere in einer Bohrung der Beleuchtungsvorrichtung 3 oder einer Schicht 18, 19 der Beleuchtungsvorrichtung 3 aufgenommen, zum Beispiel durch Einschmelzen. Der Strahlungsleiter 21 ist zum Beispiel als Glas-, Glasfaserbündel-, Kunstharz- oder Kunststoffelement ausgebildet.

Insbesondere die Figuren 3, 4, 6 und 7 zeigen, dass die Beleuchtungsvorrichtungen 3, 3A und 3B oder der Körper 6 aus mehreren Schichten 18, 19 besteht. Die Schichten 18, 19 sind vorzugsweise fest miteinander verbunden, zum Beispiel durch Verschweißen, Löten, Verschmelzen oder Verkleben.

Die Beleuchtungsvorrichtung 3 der Figuren 3 und 4 weist eine erste keramische Schicht 18 auf, in welcher bevorzugt auch der elektrische Leiter 17 (siehe Figur 5) zur Versorgung des zumindest einen Halbleiterelements 7 angeordnet ist. Vorzugsweise sind auch die elektrischen Kontakte 17A, 17B, die den elektrischen Leiter 17 mit einer elektrischen Energiequelle verbinden an der keramischen Schicht 18 vorgesehen. Die keramische Schicht 18 ist zum Beispiel rund oder kreisförmig oder ringförmig geformt.

An die keramische Schicht 18 schließt eine zweite keramische Schicht 18A an, in welcher zumindest eine Bohrung vorgesehen ist, die zumindest einen Teil der Kammer 22 bildet. Die Wand der Bohrung bildet somit einen Teil der Innenwand der Kammer 22. Daran schließt eine dritte, metallische Schicht 19 an. An dieser metallischen Schicht 19 ist bevorzugt zumindest ein Element 34 zum Anlagern und oder Befestigen der Beleuchtungsvorrichtung 3 an dem Instrumentenkopf 2 vorgesehen, zum Beispiel ein Anschlag, ein Fortsatz, ein Rücksprung, ein Vorsprung, ein Gewinde oder ähnliches. Die metallische Schicht 19 bildet auch das Strahlungsabgabeende 9 oder die Oberfläche der Beleuchtungsvorrichtung 3, an welcher sich die Licht- oder Strahlungsabgabeflächen 8 befinden.

Die drei Schichten 18, 18A und 19 sind insbesondere nicht dazu ausgebildet, sichtbares Licht zu leiten, d.h. sie sind nicht transparent. Zum Leiten der von dem zumindest einen Halbleiterelement 7 erzeugten elektromagnetische Strahlung, insbesondere des sichtbaren Lichts, ist deshalb zumindest ein im Vorstehenden bereits beschriebener optischer Strahlungsleiter 21 vorgesehen. Zur Aufnahme des Strahlungsleiters 21 weist zumindest eine der Schichten 18, 18A, 19 eine Bohrung 35 auf (zum Beispiel gemäß Figur 4 die Schicht 19), die insbesondere von dem Strahlungsabgabeende 9 oder einer Oberfläche der Beleuchtungsvorrichtung 3 bis zur Kammer 22 reicht.

Die Figur 5 zeigt in einer Aufsicht den Verlauf des elektrischen Leiters 17 zur Versorgung der Halbleiterelemente 7 mit elektrischer Energie in der keramischen Schicht 18. Es ist zu erkennen, dass die Halbleiterelemente 7 seriell geschaltet und mittels des elektrischen Leiters 17 verbunden sind. Der elektrische Leiter 17 ist des Weiteren beabstandet von dem Fluidkanal 10 angeordnet. Aufgrund der elektrischen Isolierung durch die keramische Schicht 18 benötigt der Leiter 17 keinen elektrisch isolierenden Außenmantel oder keine elektrische Außenisolierung.

Die Beleuchtungsvorrichtungen 3A und 3B der Figuren 6 und 7 ähneln in ihrem Aufbau der Beleuchtungsvorrichtung 3 der Figuren 2 - 5, wobei zur Vermeidung von Wiederholungen gleiche Bauteile mit den gleichen Bezugszeichen versehen sind.

Abweichend von der Beleuchtungsvorrichtung 3 umfasst die Beleuchtungsvorrichtung 3A der Figur 6 nur zwei Schichten 18B und 19C. Die Schicht 18B ist durch ein keramisches Material gebildet. Die Schicht 18B hat vorzugsweise die Form eines keramischen Rings. Mit der Schicht 18B verbunden ist eine metallische Schicht 19C. Die im Wesentlichen ebenfalls ringförmige Schicht 19C weist zumindest eine Bohrung 35A auf, in welcher der Strahlungsleiter 21 aufnehmbar ist. Vorzugsweise bildet zumindest ein Teil der Bohrung 35A zumindest auch einen Teil der Kammer 22 oder sind das zumindest eine Halbleiterelement 7 und / oder das Konverterelement 33 in der Bohrung 35A aufgenommen.

Die Beleuchtungsvorrichtung 3B der Figur 7 besteht ebenfalls nur aus zwei Schichten 19A, 19B. Diese Schichten 19A, 19B sind vorzugsweise beide aus Metall. Der in der Schicht 19A angeordnete elektrische Leiter 17 ist demgemäß mit einer elektrischen Isolierung versehen. In zumindest einer der beiden Schichten 19A, 19B ist wiederum eine Bohrung für den Strahlungsleiter 21 vorgesehen. In zumindest einer der beiden Schichten 19A, 19B ist eine Aufnahme für das zumindest eine Halbleiterelement 7 oder eine Bohrung, die zumindest einen Teil der Kammer 22 bildet, angeordnet.

Die Figur 8 zeigt eine Beleuchtungsvorrichtung 3C, die nicht Gegenstand des Anspruchs 1 ist, deren Aufbau im Wesentlichen den im Vorstehenden beschriebenen Beleuchtungsvorrichtung 3, 3A, 3B entspricht, so dass zur Vermeidung von Wiederholungen gleiche Bauteile wiederum mit den gleichen Bezugszeichen versehen sind. Das Strahlungsabgabeende 9 der Beleuchtungsvorrichtung 3C weist jedoch nur eine, insbesondere für sichtbares Licht, transparente Licht- oder Strahlungsabgabefläche 8 auf, die im Wesentlichen ringförmig ausgebildet ist oder sich um die Öffnung 5 erstreckt. Das Strahlungsabgabeende 9 oder die Strahlungsabgabefläche 8 ist vorzugsweise durch eine Kunststoff-, Kunstharz- oder Glasschicht 20 gebildet. Die Schicht 20 reicht entweder bis zu dem zumindest einen Halbleiterelement 7, welches somit direkt elektromagnetische Strahlung, insbesondere sichtbares Licht, an die Schicht 20 abgibt, oder zwischen der Schicht 20 und dem zumindest einen Halbleiterelement 7 ist zumindest ein optischer Strahlungsleiter, insbesondere ein Strahlungsleiter 21, wie er im Vorstehenden beschrieben wurde, vorgesehen, der die Strahlung zur Schicht 20 leitet.

In der Schicht 20 sind des Weiteren die Öffnungen 11 zur Abgabe von Fluid vorgesehen. Die Öffnungen 11 sind somit durch Strahlung abgebende oder, insbesondere für sichtbares Licht, transparente Abschnitte der Strahlungsabgabefläche 8 voneinander getrennt.

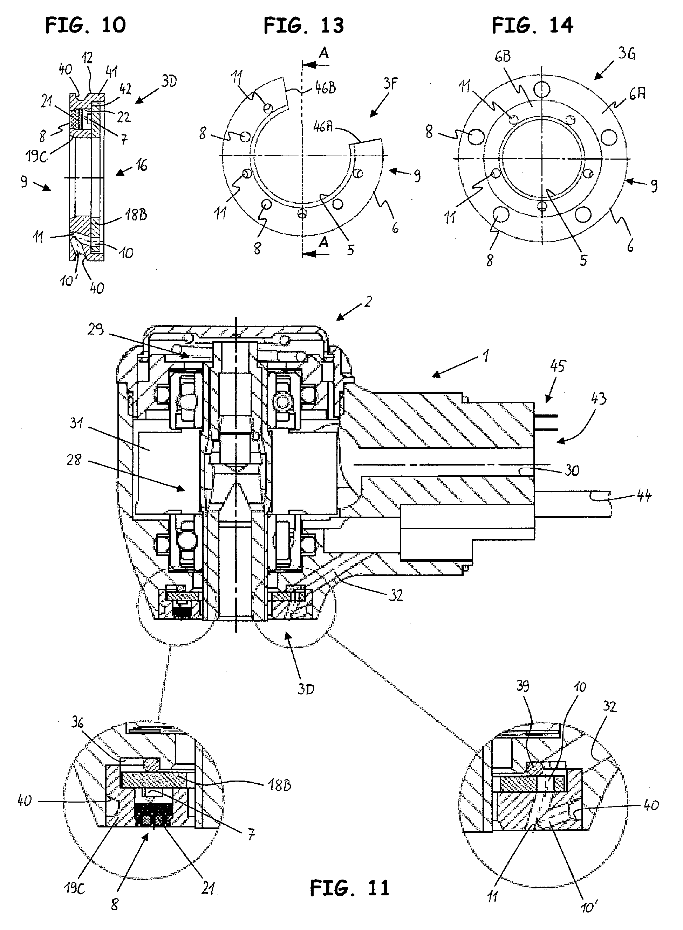

Die Beleuchtungsvorrichtung 3D der Figur 10 unterscheidet sich von den im Vorstehenden beschriebenen Beleuchtungsvorrichtungen insbesondere darin, dass an ihrer Mantelfläche 12 ein ringförmiger Kanal 40 vorgesehen ist. Der Ringkanal 40 ist zur Aufnahme eines Fluids, insbesondere von Wasser, ausgebildet und, vorzugsweise über Leitungen und / oder Kanäle im Instrument 1 mit einer Fluidquelle verbindbar oder verbunden. Der Ringkanal 40 ist mit der Fluidabgabeöffnung 11 und / oder mit der Mischkammer 13 verbunden, insbesondere über den Fluidkanal 10'. Ein derartiger Ringkanal 40 kann auch bei den Beleuchtungsvorrichtungen 3, 3A, 3B oder 3C vorgesehen sein.

Die Beleuchtungsvorrichtung 3D umfasst des Weiteren zwei Schichten, wobei bevorzugt die Schicht 18B aus Keramik und die Schicht 19C aus Metall besteht: In der Schicht 19C ist der Ringkanal 40 vorgesehen. Die Schicht 19C weist einen Flansch 41 und / oder eine Schulter 42 zur Verbindung oder Lagerung der Schicht 18B auf. Die Schulter 42 und der Ringkanal 40 sind vorzugsweise an unterschiedlichen oder gegenüber liegenden Seiten der Beleuchtungsvorrichtung 3D angeordnet. In zumindest einer Bohrung der Schicht 19C ist zumindest ein optischer Strahlungsleiter 21 aufgenommen.

Die Figur 9 zeigt eine Schnittdarstellung durch einen Instrumentenkopf 2 eines Instruments 1 mit einer Beleuchtungsvorrichtung 3A gemäß der Figur 6 (selbstverständlich sind jedoch in entsprechender Weise die Beleuchtungsvorrichtungen 3, 3B, 3C, 3D in den Instrumentenkopf 2 implementierbar). Die Beleuchtungsvorrichtung 3A ist in einer, insbesondere kreis- oder ringförmige, Aufnahme 36 oder einem Rücksprung des Instrumentenkopfs 2, vorzugsweise lösbar, angeordnet. Als Befestigungsmittel für die Beleuchtungsvorrichtung 3A ist eine Gewindehülse 37 vorgesehen, die mit einem Gewindebauteil des Instruments 1 verbindbar ist und die Beleuchtungsvorrichtung 3A in der Aufnahme 36 fixiert, zum Beispiel klemmt. Dazu sind an der Gewindehülse 37 und an der Beleuchtungsvorrichtung 3A Kontaktflächen 38A, 38B vorgesehen. Vorzugsweise ist die Gewindehülse 37 derart bemessen, dass sie in der Öffnung 5 der Beleuchtungsvorrichtung 3A aufnehmbar ist. Vorzugsweise ist die Gewindehülse 37 derart bemessen, dass zumindest ein Teil der Werkzeughaltevorrichtung 28 in der Innenbohrung der Gewindehülse 37 aufnehmbar ist.

Die Aufnahme 36 dient auch als Verbindungsstück zwischen der Medien- oder Fluidleitung 32 und dem zumindest einen Fluidkanal 10 oder als Leitungsstück, welches Fluid von der Medien- oder Fluidleitung 32 an den zumindest einen Fluidkanal 10 leitet. Insbesondere ist die Aufnahme 36 zumindest als Teil eines Kanals ausgebildet, zum Beispiel ringförmig oder als Ringkanal, der ein Fluid an mehrere Fluidkanäle 10, 10' abgibt. Vorzugsweise ist in der Aufnahme 36 ein Dichtmittel 39, zum Beispiel eine O-Ring, vorgesehen.

Schließlich ist in der Figur 9 eine Steuer- und / oder Versorgungsvorrichtung 23 dargestellt, die durch die Anschlussvorrichtung 24 mit dem Instrument 1 verbunden ist. Die Steuer- und / oder Versorgungsvorrichtung 23 versorgt das Instrument 1 über einen Versorgungsschlauch mit Medien, insbesondere Fluiden, elektrischer Energie und / oder Steuersignalen und / oder steuert oder überwacht den Betrieb des Instruments 1. Die Steuer- und / oder Versorgungsvorrichtung 23 stellt somit bevorzugt auch die elektrische Energie für das zumindest eine Halbleiterelement 7 und das zumindest eine Fluid zur Verfügung, welches über die Beleuchtungsvorrichtung 3A abgebbar ist.

Die Steuer- und / oder Versorgungsvorrichtung 23 ist vorzugsweise ausgebildet, eine alternierende Abgabe der elektromagnetischen Strahlung und des Fluids vom Licht- oder Strahlungsabgabeende 9 der Beleuchtungsvorrichtung 3A zu steuern. Dies erfolgt zum Beispiel mittels entsprechender elektrischer Steuersignale an das Halbleiterelement 7, insbesondere durch eine gepulste Versorgung des Halbleiterelements 7 mit elektrischer Energie, und durch das Öffnen und Schließen eines Steuerelements, zum Beispiel eines Ventils, in einer mit der Öffnung 11 zur Fluidabgabe verbundenen Medien- oder Fluidleitung 32.

Die Figur 11 zeigt eine Schnittdarstellung durch einen Instrumentenkopf 2 eines Instruments 1 mit einer Beleuchtungsvorrichtung 3D gemäß der Figur 10 (selbstverständlich sind jedoch in entsprechender Weise die Beleuchtungsvorrichtungen 3, 3A, 3B, 3C in den Instrumentenkopf 2 implementierbar). In den Figuren 9 und 11 dargestellte, gleiche Bauteile tragen dieselben Bezugszeichen.

Die Beleuchtungsvorrichtung 3D ist unlösbar mit dem Instrumentenkopf 2 verbunden, vorzugsweise durch Verpressen der Beleuchtungsvorrichtung 3D mit dem Instrumentenkopf 2, insbesondere durch Einpressen der Beleuchtungsvorrichtung 3D in die Aufnahme 36, oder durch eine kraftschlüssige Verbindung oder eine Reibverbindung zwischen der Beleuchtungsvorrichtung 3D und dem Instrumentenkopf 2, insbesondere der Aufnahme 36.

Um gegebenenfalls die Beleuchtungsvorrichtung 3D austauschen zu können, ist in einem bevorzugten Ausführungsbeispiel vorgesehen, dass der Instrumentenkopf 2 gemeinsam mit der Beleuchtungsvorrichtung 3D von dem Instrument 1, insbesondere von dem Griffteil 25, lösbar ist. Dazu weist der Instrumentenkopf 2 ein Kupplungselement 43 auf, das mit einem entsprechenden Kupplungselement des Griffteils 25 verbindbar ist und eine Kupplungsvorrichtung bildet. An dem Kupplungselement 43 ist zumindest eine Fluid- oder Medienleitung 30, 32, 44 vorgesehen, insbesondere für Luft oder Wasser, die mit zumindest einer entsprechenden Leitung im Griffteil 25 oder an dessen Kupplungselement, vorzugsweise mittels Stecken, verbindbar ist. An dem Kupplungselement 43 ist des Weiteren eine elektrische Verbindungsvorrichtung 45 vorgesehen, insbesondere zwei elektrische Kontakte, die mit einer entsprechenden elektrischen Verbindungsvorrichtung an dem Kupplungselement des Griffteils 25 verbindbar ist. Über diese elektrische Verbindungsvorrichtung ist die Beleuchtungsvorrichtung 3D, insbesondere das zumindest eine Halbleiterelement 7, mit einer elektrischen Energiequelle verbindbar und mit elektrischer Energie versorgbar.

Die in den Figuren 9 und 11 dargestellten Instrumente 1 sind als druckluftbetriebene Instrumente mit einem Laufrad 31 ausgebildet. Selbstverständlich ist es in entsprechender Weise möglich, eine Beleuchtungsvorrichtung 3 - 3G in ein Instrument mit einem mechanischen Antrieb des Werkzeugs 4 zu implementieren, wobei der mechanische Antrieb zumindest eine Welle umfasst, die eine Antriebsbewegung auf die Werkzeughaltevorrichtung 28 und / oder das Werkzeug 4 überträgt.

Die Figur 12 zeigt eine Beleuchtungsvorrichtung 3E, deren Körper 6 zumindest teilweise oder vollständig aus Vergussmaterial oder Spritzgussmaterial hergestellt ist, zum Beispiel aus Kunstharz, insbesondere aus Epoxidharz, oder aus einem thermoplastischen Kunststoff, zum Beispiel aus statistischen Propylen-Copolymeren (PPR), Polycarbonat (PC), Polymethylenpenten (PMP), Cyclooleofin-Copolymeren (COC) oder Polyphenylsulfon (PPSU). Vorzugsweise sind das zumindest eine Halbleiterelement 7 und / oder der zumindest eine Fluidkanal 10, 10', 40 und / oder der zumindest eine elektrische Leiter 17 und / oder die Kammer 22 und / oder der optische Strahlungsleiter 21 in das Vergussmaterial oder Spritzgussmaterial eingebettet oder von diesem zumindest teilweise umgeben. Im Übrigen entspricht die Beleuchtungsvorrichtung 3E im Wesentlichen den im Vorstehenden beschriebenen Beleuchtungsvorrichtungen 3 - 3D.

Bei den in den Figuren 1 - 12 dargestellten Beleuchtungsvorrichtungen 3 - 3E ist der Körper 6 jeweils als geschlossener Ring oder Hohlzylinder ausgebildet. Selbstverständlich besteht jedoch auch die Möglichkeit, die Beleuchtungsvorrichtung mit zwei freien Enden oder Endflächen 46A, 46B auszubilden, insbesondere gebogen oder bogenförmig oder als Kreisbogen, so wie dies beispielhaft anhand der Beleuchtungsvorrichtung 3F in der Figur 13 dargestellt ist. Zwischen den freien Enden 46A, 46B ist ein Freiraum oder eine Öffnung vorgesehen. Im Übrigen entspricht die Beleuchtungsvorrichtung 3F im Wesentlichen wiederum den im Vorstehenden beschriebenen Beleuchtungsvorrichtungen 3 - 3E. Selbstverständlich besteht auch die Möglichkeit, die Beleuchtungsvorrichtung eckig geschlossen oder eckig mit freien Enden (gewinkelt) auszubilden.

Die Figur 14 zeigt eine Beleuchtungsvorrichtung 3G, deren Körper 6 aus zwei Teilen 6A, 6B besteht, wobei an einem Teil 6A das Strahlungsabgabeende 9 und / oder die zumindest eine Strahlungsabgabefläche 8 und an dem anderen Teil 6B die zumindest eine Öffnung 11 zur Abgabe von Fluid vorgesehen ist. Vorzugsweise sind an einem der beiden Teile 6A, 6B, insbesondere an jenem Teil 6A mit dem Strahlungsabgabeende 9 und / oder der zumindest einen Strahlungsabgabefläche 8, das zumindest eine Halbleiterelement 7 und / oder der zumindest eine elektrische Leiter 17 und / oder die Kammer 22 und / oder der optische Strahlungsleiter 21 vorgesehen. Alternativ oder zusätzlich sind vorzugsweise an einem der beiden Teile 6A, 6B, insbesondere an jenem Teil 6B mit der zumindest einen Öffnung 11 zur Abgabe von Fluid der zumindest eine Fluidkanal 10, 10', 40 vorgesehen. Vorzugsweise umgibt ein Teil 6A, 6B den anderen Teil 6A, 6B. Vorzugsweise sind die beiden Teile 6A, 6B konzentrisch angeordnet. Vorzugsweise sind die beiden Teile 6A, 6B unlösbar miteinander verbunden. Vorzugsweise sind die beiden Teile 6A, 6B aus gleichem oder unterschiedlichem Material hergestellt. Im Übrigen entspricht die Beleuchtungsvorrichtung 3G im Wesentlichen den im Vorstehenden beschriebenen Beleuchtungsvorrichtungen 3 - 3F.

## Patentansprüche

1. Beleuchtungsvorrichtung (3 - 3G) für einen Instrumentenkopf (2) eines medizinischen, insbesondere dentalen, Instruments (1), wobei an dem Instrumentenkopf (2) ein Werkzeug (4) zum Einwirken auf eine Behandlungsstelle vorgesehen ist und wobei die Beleuchtungsvorrichtung (3 - 3G) umfasst: Eine Aufnahme oder Öffnung (5), in welcher das Werkzeug (4) aufnehmbar ist, einen die Aufnahme oder Öffnung (5) umgebenden Körper (6), zumindest ein am Körper (6) vorgesehenes Halbleiterelement (7), das zur Emission von elektromagnetischer Strahlung ausgebildet ist, eine oder mehrere optisch leitende, insbesondere transparente, Strahlungsabgabeflächen (8) an einem Strahlungsabgabeende (9) des Körpers (6) der Beleuchtungsvorrichtung (3 - 3G), über welche die von dem zumindest einen Halbleiterelement (7) emittierte elektromagnetische Strahlung von der Beleuchtungsvorrichtung (3 - 3G) abgebbar ist, und zumindest einen in dem Körper (6) der Beleuchtungsvorrichtung (3 - 3G) vorgesehenen Fluidkanal (10, 10', 40), der zumindest eine am Strahlungsabgabeende (9) der Beleuchtungsvorrichtung (3 - 3G) vorgesehene Öffnung (11) zur Abgabe von Fluid mit einer oder mehreren Fluidquellen verbindet, **dadurch gekennzeichnet, dass**
die zumindest eine Öffnung (11) zur Abgabe von Fluid und die eine oder mehreren Strahlungsabgabeflächen (8) durch einen Bereich voneinander beabstandet sind, der keine elektromagnetische Strahlung, insbesondere keine sichtbare elektromagnetische Strahlung, und kein Fluid abgibt.

2. Beleuchtungsvorrichtung (3 - 3G) nach Anspruch 1, **dadurch gekennzeichnet, dass** der Körper (6) der Beleuchtungsvorrichtung (3 - 3G) eine sich von dem Strahlungsabgabeende (9) erstreckende Mantelfläche (12) aufweist, welche einen in sich geschlossenen, insbesondere ringförmigen oder mehreckigen, Außenumfang bildet, innerhalb dem der zumindest eine Fluidkanal (10, 10', 40) angeordnet ist.

3. Beleuchtungsvorrichtung (3 - 3G) nach Anspruch 2, **dadurch gekennzeichnet, dass** des Weiteren die eine oder mehreren optisch leitenden Strahlungsabgabeflächen (8) und die zumindest eine Öffnung (11) zur Abgabe von Fluid innerhalb des in sich geschlossenen, insbesondere ringförmigen oder mehreckigen, durch die Mantelfläche (12) gebildeten Außenumfangs angeordnet sind.

4. Beleuchtungsvorrichtung (3 - 3G) nach einem der Ansprüche 1 - 3, **gekennzeichnet durch** mehrere in dem Körper (6) der Beleuchtungsvorrichtung (3 - 3G) vorgesehene Fluidkanäle (10, 10', 40), die in eine gemeinsame Fluidmischkammer (13) in der Beleuchtungsvorrichtung (3 - 3G) münden, wobei die Fluidmischkammer (13) mit der zumindest einen Öffnung (11) zur Abgabe von Fluid verbunden ist.

5. Beleuchtungsvorrichtung (3 - 3G) nach einem der Ansprüche 2 - 4, **gekennzeichnet durch** mehrere in dem Körper (6) der Beleuchtungsvorrichtung (3 - 3G) vorgesehene Fluidkanäle (10, 10', 40), wobei zumindest ein Fluidkanal (10') eine Öffnung (14) zur Verbindung mit einer Fluidquelle an der Mantelfläche (12) des Körpers (6) der Beleuchtungsvorrichtung (3 - 3G) und zumindest ein Fluidkanal (10) eine Öffnung (15) zur Verbindung mit einer Fluidquelle an einem Instrumentenanschlussende (16) des Körpers (6) der Beleuchtungsvorrichtung (3 - 3G) aufweist.

6. Beleuchtungsvorrichtung (3 - 3G) nach einem der Ansprüche 1 - 5, **gekennzeichnet durch** mehrere optisch leitende Strahlungsabgabefläche (8) und mehrere Öffnungen (11) zur Abgabe von Fluid, wobei zwischen zwei Öffnungen (11) zur Abgabe von Fluid zumindest eine Strahlungsabgabefläche (8) angeordnet ist.

7. Beleuchtungsvorrichtung (3 - 3G) nach einem der Ansprüche 1 - 6, **dadurch gekennzeichnet, dass** mehrere elektrisch seriell geschaltete, mittels eines elektrischen Leiters (17) verbundene Halbleiterelemente (7) vorgesehen sind, wobei der elektrische Leiter (17) beabstandet von dem zumindest einen in dem Körper (6) der Beleuchtungsvorrichtung (3 - 3G) vorgesehenen Fluidkanal (10, 10', 40) angeordnet ist.

8. Beleuchtungsvorrichtung (3 - 3G) nach einem der Ansprüche 1 - 7, **dadurch gekennzeichnet, dass** die Beleuchtungsvorrichtung (3 - 3G) aus mehreren Schichten (18, 19), insbesondere aus mehreren Schichten (18, 19) unterschiedlicher Materialien, aufgebaut ist.

9. Beleuchtungsvorrichtung (3 - 3G) nach Anspruch 8, **dadurch gekennzeichnet, dass** zumindest eine der Schichten (18, 18A, 18B) ein keramisches Material und / oder Kunststoff umfasst, insbesondere jene Schicht, in welcher das zumindest eine Halbleiterelement (7) und / oder ein elektrischer Leiter (17) zur Versorgung des zumindest einen Halbleiterelements (7) mit elektrischer Energie vorgesehen ist / sind.

10. Beleuchtungsvorrichtung (3 - 3G) nach Anspruch 8 oder 9, **dadurch gekennzeichnet, dass** zumindest eine der Schichten metallisches Material (19, 19A, 19B, 19C) und / oder Kunststoff und / oder Glas (20) umfasst, insbesondere jene Schicht, die das Strahlungsabgabeende (9) der Beleuchtungsvorrichtung (3 - 3G) bildet.

11. Beleuchtungsvorrichtung (3 - 3G) nach einem der Ansprüche 1 - 10, **dadurch gekennzeichnet, dass** mehrere Halbleiterelemente (7) vorgesehen sind, wobei jedem Halbleiterelement (7) ein eigener optischer Strahlungsleiter (21) zugeordnet ist, der derart angeordnet ist, dass er die von den Halbleiterelementen (7) emittierte elektromagnetische Strahlung in Richtung des Strahlungsabgabeendes (9) der Beleuchtungsvorrichtung (3 - 3G) leitet.

12. Beleuchtungsvorrichtung (3 - 3G) nach einem der Ansprüche 1 - 11, **dadurch gekennzeichnet, dass** das zumindest eine Halbleiterelement (7) in einer, insbesondere hermetisch verschlossenen, Kammer (22) im Körper (6) der Beleuchtungsvorrichtung (3 - 3G) angeordnet ist.

13. Beleuchtungsvorrichtung (3 - 3G) nach einem der Ansprüche 1 - 12, **gekennzeichnet durch** eine Steuervorrichtung (23) zur alternierenden Abgabe der elektromagnetischen Strahlung und des Fluids vom Strahlungsabgabeende (9) der Beleuchtungsvorrichtung (3 - 3G).

14. Beleuchtungsvorrichtung (3 - 3G) nach einem der Ansprüche 1 - 13, **gekennzeichnet, durch** einen Ringkanal (40) an einer Mantelfläche (12) der Beleuchtungsvorrichtung (3 - 3G) zur Aufnahme eines Fluids, insbesondere von Wasser, wobei der Ringkanal (36, 40) mit der zumindest einen Öffnung (11) zur Abgabe von Fluid der Beleuchtungsvorrichtung (3 - 3G) verbunden ist.

15. Instrumentenkopf (2) eines medizinischen, insbesondere dentalen, Instruments (1), **gekennzeichnet durch** eine Beleuchtungsvorrichtung (3 - 3G) gemäß einem der Ansprüche 1 - 14.

## Claims

1. A lighting device (3 - 3G) for an instrument head (2) of a medical, in particular dental, instrument (1), wherein a tool (4) for acting on a treatment site is provided on the instrument head (2), and wherein the lighting device (3 - 3G) comprises: a receptacle or an opening (5), in which the tool (4) can be accommodated, a body (6) surrounding the receptacle or opening (5), at least one semiconductor element (7) provided on the body (6), designed for emission of electromagnetic radiation, one or more optically-conducting, in particular transparent radiation-emitting surfaces (8) on a radiation-emitting end (9) of the body (6) of the lighting device (3 - 3G), by means of which the electromagnetic radiation emitted by the at least one semiconductor element (7) can be emitted by the lighting device (3 - 3G), and at least one fluid channel (10, 10', 40) which is provided in the body (6) of the lighting device (3 - 3G) and connects at least one opening (11) for dispensing fluid, provided on the radiation-emitting end (9) of the lighting device (3 - 3G), to one or more fluid sources, **characterized in that** the at least one opening (11) for dispensing fluid and the one or more radiation-emitting surfaces (8) are spaced a distance apart by a region which does not emit any electromagnetic radiation, in particular any visible electromagnetic radiation, and does not dispense any fluid.

2. The lighting device (3 - 3G) according to Claim 1, **characterized in that** the body (6) of the lighting device (3 - 3G) comprises a lateral surface (12) extending from the radiation-emitting end (9), which forms a self-contained, in particular ring-shaped or polygonal exterior circumference within which the at least one fluid channel (10, 10', 40) is arranged.

3. The lighting device (3 - 3G) according to Claim 2, **characterized in that** further the one or more optically-conducting radiation-emitting surfaces (8) and the at least one opening (11) for dispensing fluid are also arranged within the self-contained, in particular annular or polygonal exterior circumference formed by the lateral surface (12).

4. The lighting device (3 - 3G) according to any one of Claims 1 - 3, **characterized by** a plurality of fluid channels (10, 10', 40) which are provided in the body (6) of the lighting device (3 - 3G) and which open into a shared fluid mixing chamber (13) in the lighting device (3 - 3G), wherein the fluid mixing chamber (13) is connected to the at least one opening (11) for dispensing fluid.

5. The lighting device (3 - 3G) according to any one of Claims 2 - 4, **characterized by** a plurality of fluid channels (10, 10', 40) provided in the body (6) of the lighting device (3 - 3G), wherein at least one fluid channel (10') comprises an opening (14) for connection to a fluid source on the lateral surface (12) of the body (6) of the lighting device (3 - 3G) and at least one fluid channel (10) comprises an opening (15) for connection to a fluid source on an instrument-connecting end (16) of the body (6) of the lighting device (3 - 3G).

6. The lighting device (3 - 3G) according to any one of Claims 1 - 5, **characterized by** a plurality of optically-conducting radiation-emitting surfaces (8) and a plurality of openings (11) for dispensing fluid, wherein at least one radiation-emitting surface (8) is arranged between two openings (11) for dispensing fluid.

7. The lighting device (3 - 3G) according to any one of Claims 1 - 6, **characterized in that** a plurality of electrically series-connected semiconductor elements (7) which are connected by means of an electric conductor (17) are provided, wherein the electric conductor (17) is arranged at a distance from the at least one fluid channel (10, 10', 40) provided in the body (6) of the lighting device (3 - 3G).

8. The lighting device (3 - 3G) according to any one of Claims 1 - 7, **characterized in that** the lighting device (3 - 3G) is constructed of multiple layers (18, 19), in particular multiple layers (18, 19) of different materials.

9. The lighting device (3 - 3G) according to Claim 8, **characterized in that** at least one of the layers (18, 18A, 18B) comprises a ceramic material and/or plastic, in particular the layer in which the at least one semiconductor element (7) and/or an electric conductor (17) for supplying the at least one semiconductor element (7) with electric power is/are provided.

10. The lighting device (3 - 3G) according to Claim 8 or 9, **characterized in that** at least one of the layers comprises metallic material (19, 19A, 19B, 19C) and/or plastic and/or glass (20), in particular the layer that forms the radiation-emitting end (9) of the lighting device (3 - 3G).

11. The lighting device (3 - 3G) according to any one of Claims 1 - 10, **characterized in that** a plurality of semiconductor elements (7) is provided, wherein each semiconductor element (7) being assigned its own optical radiation conductor (21) which is arranged so that it conducts the electromagnetic radiation emitted by the semiconductor elements (7) in the direction of the radiation-emitting end (9) of the lighting device (3 - 3G).

12. The lighting device (3 - 3G) according to any one of Claims 1 - 11, **characterized in that** the at least one semiconductor element (7) is arranged in a, in particular hermetically sealed, chamber (22) in the body (6) of the lighting device (3 - 3G).

13. The lighting device (3 - 3G) according to any one of Claims 1 - 12, **characterized by** a control unit (23) for alternating emission of the electromagnetic radiation and dispensing of the fluid from the radiation-emitting end (9) of the lighting device (3 - 3G).

14. The lighting device (3 - 3G) according to any one of Claims 1 - 13, **characterized by** a ring-shaped channel (40) for receiving a fluid, in particular water, on a lateral surface (12) of the lighting device (3 - 3G), wherein the ring-shaped channel (36, 40) is connected to the at least one opening (11) for dispensing fluid of the lighting device (3 - 3G).

15. An instrument head (2) of a medical, in particular dental, instrument (1), **characterized by** a lighting device (3 - 3G) according to any one of Claims 1 - 14.

## Revendications

1. Dispositif d'éclairage (3 - 3G) pour une tête d'instrument (2) d'un instrument médical (1), en particulier dentaire, étant prévu sur la tête d'instrument (2) un outil (4) permettant d'agir sur un point de traitement et le dispositif d'éclairage (3 - 3G) comprenant: une porte ou orifice (5) dans lequel l'outil (4) peut être reçu, un corps (6) entourant la porte ou l'orifice (5), au moins un élément semi-conducteur (7) prévu sur le corps (6) et qui est conçu pour émettre un rayonnement électromagnétique, une ou plusieurs surfaces de émission de rayons (8) conductrices optiques, en particulier transparentes, à une extrémité émettrice de rayons (9) du corps (6) du dispositif d'éclairage (3 - 3G) par l'intermédiaire desquelles le rayonnement électromagnétique émis par l'au moins un élément semi-conducteur (7) peut être diffusé par le dispositif d'éclairage (3 - 3G) et au moins un canal à fluide (10, 10', 40) prévu dans le corps (6) du dispositif d'éclairage (3 - 3 G) et qui relie au moins un orifice (11) de diffusion de fluide prévu à l'extrémité émettrice de rayons (9) du dispositif d'éclairage (3 - 3G) avec une ou plusieurs sources de fluide, **caractérisé en ce que**
l'au moins un orifice (11) de diffusion de fluide et l'une ou les plusieurs surfaces de émission de rayons (8) sont espacés par une zone qui ne diffuse pas de rayonnement électromagnétique, en particulier pas de rayonnement électromagnétique visible, et pas de fluide.

2. Dispositif d'éclairage (3 - 3 G) selon la revendication 1, **caractérisé en ce que** le corps (6) du dispositif d'éclairage (3 - 3G) présente une surface d'enveloppe (12) s'étendant depuis l'extrémité émettrice de rayons (9) et qui constitue une surface extérieure refermée sur elle-même, en particulier de forme circulaire ou polygonale, dans laquelle l'au moins un canal à fluide (10, 10', 40) est disposé.

3. Dispositif d'éclairage (3 - 3 G) selon la revendication 2, **caractérisé en ce que** l'une ou les plusieurs surfaces de émission de rayons (8) conductrices optiques et l'au moins un orifice (11) de diffusion de fluide sont disposés dans la circonférence extérieure refermée sur elle-même, en en particulier de forme circulaire ou polygonale, constituée par la surface d'enveloppe (12).

4. Dispositif d'éclairage (3 - 3 G) selon une des revendications 1 à 3, **caractérisé par** plusieurs canaux à fluide (10, 10', 40) prévus dans le corps (6) du dispositif d'éclairage (3 - 3 G), qui débouchent dans une chambre de mélange de fluide commune (13) dans le dispositif d'éclairage (3 - 3G), la chambre de mélange de fluide (13) étant raccordée à l'au moins un orifice (11) de diffusion de fluide.

5. Dispositif d'éclairage (3 - 3G) selon une des revendications 2 à 4, **caractérisé par** plusieurs canaux à fluide (10, 10', 40) prévus dans le corps (6) du dispositif d'éclairage (3 - 3 G), au moins un canal à fluide (10') présentant un orifice (14) à raccorder à une source de fluide au niveau de la surface d'enveloppe (12) du corps (6) du dispositif d'éclairage (3 - 3G) et au moins un canal à fluide (10) présentant un orifice (15) à raccorder à une source de fluide au niveau d'une extrémité de raccordement d'instruments (16) du corps (6) du dispositif d'éclairage (3 - 3G).

6. Dispositif d'éclairage (3 - 3 G) selon une des revendications 1 à 5, **caractérisé par** plusieurs surfaces de émission de rayons (8) conductrices optiques et plusieurs orifices (11) de diffusion de fluide, au moins une surface de émission de rayons (8) étant disposée entre deux orifices (11) de diffusion de fluide.

7. Dispositif d'éclairage (3 - 3G) selon une des revendications 1 à 6, **caractérisé en ce que** sont prévus plusieurs éléments semi-conducteurs (7) branchés en série et reliés au moyen d'un conducteur électrique (17), le conducteur électrique (17) étant disposé espacé de l'au moins un canal à fluide (10, 10', 40) prévu dans le corps (6) du dispositif d'éclairage (3 - 3G).

8. Dispositif d'éclairage (3 - 3G) selon une des revendications 1 à 7, **caractérisé en ce que** le dispositif d'éclairage (3 - 3G) est composé de plusieurs couches (18, 19), en particulier plusieurs couches (18, 19), de matériaux différents.

9. Dispositif d'éclairage (3 - 3 G) selon la revendication 8, **caractérisé en ce que** qu'au moins une des couches (18, 18A, 18B) comprend un matériau céramique et/ou du plastique, en particulier la couche dans laquelle l'au moins un élément semi-conducteur (7) et/ou un conducteur électrique (17) pour alimenter l'au moins un élément semi-conducteur (7) en énergie électrique est/sont prévu(s).

10. Dispositif d'éclairage (3 - 3G) selon la revendication 8 ou 9, **caractérisé en ce que** au moins une des couches comprend du matériau métallique (19, 19A, 19B, 19C) et/ou du plastique et/ou du verre (20), en particulier la couche qui constitue l'extrémité émettrice de rayons (9) du dispositif d'éclairage (3 - 3G).

11. Dispositif d'éclairage (3 - 3G) selon une des revendications 1 à 10, **caractérisé en ce que** sont prévus plusieurs éléments semi-conducteurs (7), étant associé à chaque élément semi-conducteur (7) son propre conducteur optique de rayons (21) qui est disposé de manière à conduire le rayonnement électromagnétique émis par les éléments semi-conducteurs (7) en direction de l'extrémité émettrice de rayons (9) du dispositif d'éclairage (3 - 3G).

12. Dispositif d'éclairage (3 - 3G) selon une des revendications 1 à 11, **caractérisé en ce que** l'au moins un élément semi-conducteur (7) est disposé dans une chambre (22), en particulier fermée hermétiquement, du corps (6) du dispositif d'éclairage (3 - 3G).

13. Dispositif d'éclairage (3 - 3 G) selon une des revendications 1 à 12, **caractérisé par** un dispositif de commande (23) pour la diffusion en alternance du rayonnement électromagnétique et du fluide par l'extrémité émettrice de rayons (9) du dispositif d'éclairage (3 - 3G).

14. Dispositif d'éclairage (3 - 3 G) selon une des revendications 1 à 13, **caractérisé par** un canal annulaire (40) sur une surface d'enveloppe (12) du dispositif d'éclairage (3 - 3G) pour recevoir un fluide, en particulier de l'eau, le canal annulaire (36, 40) étant raccordé à l'au moins un orifice (11) de diffusion de fluide du dispositif d'éclairage (3 - 3G).

15. Tête d'instrument (2) d'un instrument médical (1), en particulier dentaire, **caractérisée par** un dispositif d'éclairage (3 - 3 G) selon une des revendications 1 à 14.
